Europäisches Patentamt

European Patent Office

Office européen des brevets

(11) Publication number: **0 181 199**
**A2**

(12) **EUROPEAN PATENT APPLICATION**

(21) Application number: **85308057.0**

(22) Date of filing: **06.11.85**

(51) Int. Cl.⁴: **A 61 B 17/36**

(30) Priority: **09.11.84 GB 8428411**

(43) Date of publication of application:
**14.05.86 Bulletin 86/20**

(84) Designated Contracting States:
**AT BE CH DE FR GB IT LI LU NL SE**

(71) Applicant: **MICRA Ltd.**
**54 Alma Street**
**Luton Bedfordshire LU1 2PL(GB)**

(72) Inventor: **Hoskin, William John**
**5 Long Buftlers**
**Harpenden Hertfordshire AL5 1JF(GB)**

(74) Representative: **Enskat, Michael Antony Frank et al,**
**Saunders & Dolleymore 2 Norfolk Road**
**Rickmansworth Hertfordshire WD3 1JH(GB)**

(54) **Laser knives.**

(57) A laser knife includes a laser (5) supplying a diamond blade (4) with cauterising radiation. A fluid supply (9) supplies fluid to the blade (4) between the blade and the tissue being severed to reduce the tendency of the blade of becoming encrusted with burnt or charred tissue or other debris.

FIG.1

EP 0 181 199 A2

LASER KNIVES

The present invention relates to laser knives.

Surgical scalpels have been proposed having a sapphire blade. An argon laser is coupled to the blade and energised to inject its high intensity light output into the immediately adjacent region of tissue being incised. The blue green light of the laser is selectively absorbed by the red haemoglobin of the blood in the walls of the incised tissue and rapid coagulation occurs to seal or cauterise the walls of the incision. Also the blade becomes heated.

One problem exhibited by such scalpels is that when the heated blade comes into contact with a cauterised tissue this tissue tends to adhere to the blade and this reduces the efficiency of the cutting action and increases the need for more frequent servicing of the knife. However, it can be shown that this adhesion can be avoided either by operating the blade at a very high temperature or at a very low temperature. If the blade is operated at a high temperature and if the blade is heated only by the laser energy then that amount of energy projected forward from the blade can be sufficiently great to penetrate far deeper and cauterise and seal tissue which one would not wish to seal. Under these circumstances it is the power which is adjusted to provide the right temperature for a blade, and not the temperature to provide optimum depth of cauterisation.

Thus, it is an object of the invention to provide an improved laser blade.

According to the present invention there is provided a laser knife comprising a transparent blade, means for transmitting a cauterising radiation through the blade to effect cauterisation of the tissue severed by the blade, and means for maintaining the tissue spaced from the blade once severed by the blade.

According to the present invention there is further provided a laser knife for simultaneously severing and cauterising tissue, the knife including a blade and means for directing a layer of fluid over the blade to maintain the blade substantially free from encrustation by burnt or charred tissue or other debris.

A laser knife embodying the invention will now be described, by way of example only, with reference to the accompanying diagrammatic drawings in which:

Figure 1 is a plan view of a laser knife;

Figure 2 is a cross-section to an enlarged scale of the blade assembly of the knife; and

Figure 3 is a cross-section to an enlarged scale of a modified blade for the blade assembly of Figures 1 and 2.

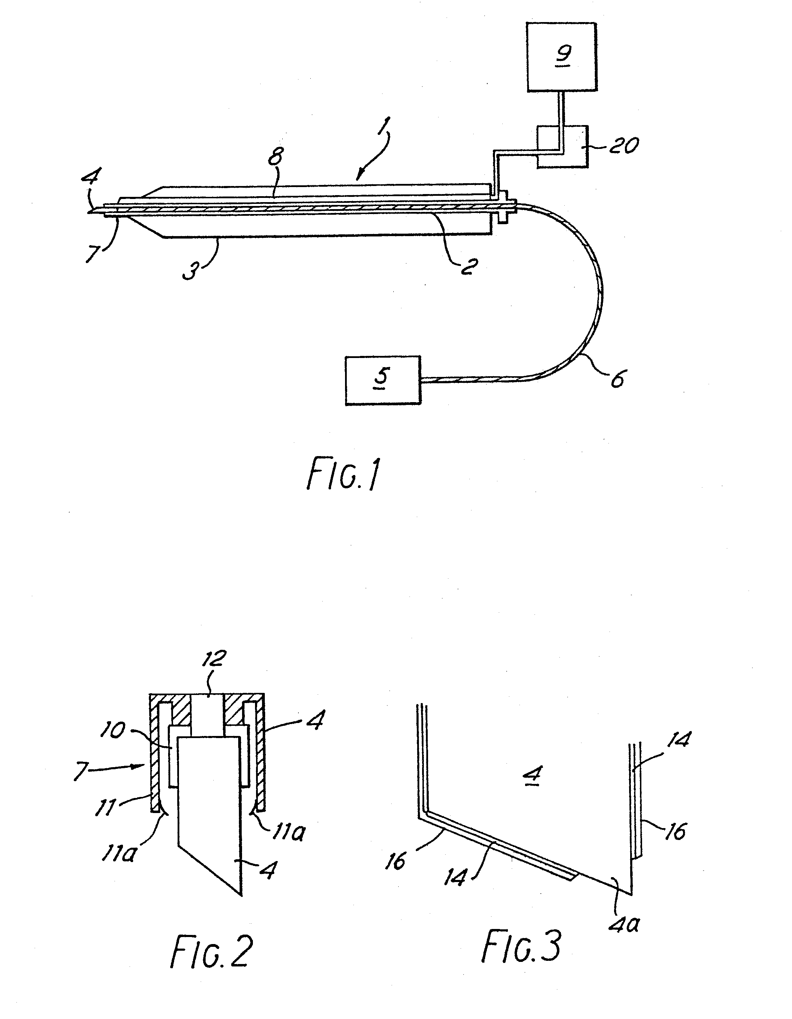

As shown in Figure 1, a surgical diamond knife 1 has a handle 3 supporting a diamond blade 4. An optical fibre bundle 2 located within the handle 3 abuts an optically smooth surface of the diamond blade 4. A connector 6 couples the other end of the optical fibre bundle 3 to a radiation source in the form of a Neodymium/Yttrium Aluminium Garnet (Nd/YAG) Laser 5.

A shield assembly 7, for directing a stream of water towards the cutting edge of the blade 4, supports the blade 4 on the handle 3. A conduit 8 within the handle couples the shield assembly 7 to a source 9 of water under pressure.

The shield assembly 7 as shown more clearly in Figure 2 includes an inner support member 10 which supports the blade 4 and an outer member 11 which together with the inner member 10 defines a channel through which water can be directed towards the cutting edge of the blade 4. Deflectors 11a are mounted on the inner face of the member 11 to provide a constriction in the channel and to deflect the flow of water towards the blade 4.

The outer member 10 has a central through hole which is filled by an optical coupling member 12 for coupling radiation from the optical fibre bundle 3 to the blade 4.

The Nd/YAG laser 5 is in the form of a crystal supplied by a continuous source of power. Instead the crystal can be pumped by means of a flash tube which provides a pulsed source of power.

To effect coagulation the laser is operated at a constant output (e.g. 2-15 watts output).

With the above-described specific arrangement, i.e. the combination of a diamond blade and Nd/YAG laser, it has been found that the blade has a significantly longer life when compared with existing arrangements of radiation transmitting blades and lasers. It will be appreciated that instead of diamond blades, other transparent hard substances such as sapphire can be used.

It has been found that the adhesion of the material being cut to the blade appears to effect the cutting efficiency of the blade.

The function of the water stream directed onto the blade 4 by the shield assembly 7 is to cool the blade and/or to tend to maintain a separation between the severed tissue and the blade. In this way the tendency of any burnt, dried or charred tissue or debris to adhere to the blade is reduced to a minimum.

The use of a diamond blade provides the cutting edge with a relatively long life. This is due to the fact that diamond is extremely strong material and because of this it is possible to grind the edge such that the radius of curvature at the edge is 10 nanometers. This is approximately 3 to 4 atoms thick. This very fine edge cannot be readily produced on other materials since the internal forces are such that the stress induced by such a fine edge would cause such an edge in other materials to shatter. Additional stress caused by thermal shock can further deteriorate the cutting edge of such other materials, and hence the diamond is far superior to materials such as sapphire, quartz or glass for this reason alone.

With this arrangement it has been found that the periods between successive maintenance services can be significantly increased and furthermore the knife blade cuts and cauterises more efficiently.

Instead of water pumped to the shield assembly, a saline solution can be used.

Preferably the water or saline solution are cooled by a cooler 20 to well below blood temperature before being supplied to the shield assembly.

The laser knife of the present invention may, optionally, be combined with a conventional visible light source to cause the blade to luminesce (as disclosed in co-pending UK patent specification 2,102,678. This combination is particularly useful when the knife is used, e.g. in brain surgery, where most incisions are made through small holes which may be deep and therefore dark.

The cauterising action of the blade is particularly valuable in removing cancerous tissue. When cutting away such tissue the surfaces being incised are speedily sealed against fluid loss an they are traversed by the cutting edge of the blade. The free flow of body fluids is thus prevented and the chances of recovery from the operation significantly improved.

The free flow of water emerging from the shield helps to remove and loosen debris from the cauterised tissue to leave a clean wound.

In a modification radiation from the laser may be coupled from the laser to the diamond blade by means of alternative methods such as those disclosed in co-pending Application No. 81 23635 (Published Specification No. 2 102 678).

In a modification the transparent blade surface is coated with a sterilisable non-stick or low friction coating for example TEFLON (Registered Trade Mark).

The coating does not of course cover the cutting edge of the blade so as to allow the laser radiation to escape from the blade.

The adhesion of the TEFLON to the blade may be assisted by first plating the blade surfaces with a layer of gold.

Figure 3 shows the blade 4 in more detail. As shown, the blade 4 is coated with a layer 14 of gold which in turn is coated with a layer 16 of TEFLON (Registered Trade Mark). The cutting edge portion 4a of the blade 4 remains uncoated.

2385EP
0181199

CLAIMS

1.        A laser knife comprising a transparent blade, means for transmitting a cauterising radiation through the blade to effect cauterisation of the tissue severed by the blade, and means for maintaining the tissue spaced from the blade once severed by the blade.

2.        A laser knife according to Claim 1 wherein the spacing means comprises means for introducing a layer of water between the blade and the tissue.

3.        A laser knife for simultaneously severing and cauterising tissue, the knife including a blade and means for directing a layer of fluid over the blade to maintain the blade substantially free from encrustation by burnt or charred tissue or other debris.

4.        A laser knife according to Claim 3 wherein the said means comprises a shield supporting said blade and being so profiled as to direct said fluid from a source under pressure over said blade.

5.        A laser knife according to any preceding claim comprising a diamond blade, a Neodymium/Yttrium Aluminium Garnet laser and means optically coupling the radiation output from the vicinity of the cutting edge of the blade to enable the blade to cauterise the tissue severed by the knife.

6.        A laser knife according to any preceding claim wherein the blade except for the cutting edge thereof is coated with a low friction material.

7.        A laser knife according to Claim 6 wherein there is a layer of gold between the low friction material and the blade.

8.        A laser knife according to any preceding claim wherein the blade comprises a diamond having a cutting edge which has a radius of curvature of less than 10 microns.

9.      A laser knife according to Claim 2 or to Claim 3 including cooling means for colling the water of fluid.

FIG.1

FIG.2

FIG.3